# EUROPÄISCHE PATENTSCHRIFT

(11) **EP 1 457 170 B1**
(45) Veröffentlichungstag und Bekanntmachung des Hinweises auf die Patenterteilung: **07.12.2005**
(21) Anmeldenummer: 04005529.5
(22) Anmeldetag: 09.03.2004
(51) Int. Cl.: A61F 2/16

(54) **Hinterkammerlinse**
Posterior chamber intraocular lens
Lentille intraoculaire de chambre postérieure

(30) Priorität: 13.03.2003 DE 10310961
(43) Veröffentlichungstag der Anmeldung: 15.09.2004
(73) Patentinhaber: Aixmed GmbH, 52222 Stolberg (DE)
(72) Erfinder: Jansen, Matthias, Dipl.-Phys., 52223 Stolberg (DE)
(74) Vertreter: Bauer, Dirk

(56) Entgegenhaltungen:
- WO-A-99/03427
- WO-A-03/015668
- US-A- 5 653 754

## Beschreibung

Die Erfindung betrifft eine Hinterkammerlinse, die anstelle einer natürlichen Linse eines Auges in einen Kapselsack der Linse derart implantierbar ist, dass eine optische Achse der Hinterkammerlinse eine Netzhaut des Auges trifft, wobei die Hinterkammerlinse einen Linsenkörper mit einer zu der optischen Achse senkrechten Hauptebene und mehrere Haptiken aufweist, die jeweils in einem Anschlussbereich an den Linsenkörper anschließen und die mittels im Wesentlichen starrer, in der Hauptebene von dem Linsenkörper abstehender Stützfüße dessen Abstützung in dem Kapselsack gewährleisten.

Mittels Intraokularlinsen (auch: IOL, Implantlinse, Pseudophakos oder künstliche Augenlinse), also physiologisch neutralen Optikplastiken, sind Brechungsfehler eines Auges korrigierbar. Ein solcher Brechungsfehler kann sowohl durch eine krankhafte Fehlbildung als auch durch die operative Entfernung der natürlichen Augenlinse verursacht sein. Zur Korrektur einer Linsenlosigkeit (Aphakie) wird der mikrochirurgische Eingriff der Implantation einer Intraokularlinse heutzutage sowohl gegenüber Kontaktlinsen als auch anstelle von Starbrillen deutlich bevorzugt. Aber auch Kurz- oder Weitsichtigkeit kann mittels einer Intraokularlinse, einer sogenannten "implantierten Kontaktlinse" (ICL), reduziert werden, wobei die körpereigene Linse - soweit keine Alterssichtigkeit vorliegt - die Fähigkeit zur Naheinstellung (Akkomodation) behält.

Eine spezielle Form der Intraokularlinse stellt die kapselsackfixierte Hinterkammerlinse dar, die anstelle einer natürlichen Linse in einen Kapselsack implantiert wird. Beispielsweise im Rahmen der operativen Korrektur einer Starerkrankung (Katarakt) wird zunächst der getrübte natürliche Linsenkörper entfernt. Durch eine Inzision in der Hornhaut wird die bindegewebige Linsenkapsel kreisförmig eröffnet und ihr vorderer Teil sowie die faserige Rinde und der Kern der Linse entfernt, so dass nur die intakte hintere Linsenkapsel und seitliche Teile der vorderen Linsenkapsel zurückbleiben. Die Reste der Linsenkapsel formen einen nach vorne offenen schlaffen "Kapselsack", in den der Operateur die Hinterkammerlinse einsetzt und mittels ihrer Haptiken verankert.

Hinterkammerlinsen weisen typischer Weise einen Linsenradius zwischen 2 und 4,5 mm, die einander gegenüber liegenden Stützflächen der Haptiken eine Abstand zwischen 10 und 15 mm auf.

In der US 5,197,981 A ist beispielsweise eine Hinterkammerlinse vorgeschlagen, die mit zwei fadenförmigen Haptiken in einem Arbeitsschritt aus Plexiglas (Polymethylmetacrylat) gespritzt wird. Derartige Hinterkammerlinsen mit starrem Linsenkörper erfordern eine Inzision in die Hornhaut, die dem Durchmesser des Linsenkörpers entspricht. Prinzipiell können Hinterkammerlinsen aus jedem Material bestehen, dass einerseits biokompatibel ist und andererseits geeignete optische Eigenschaften aufweist.

Da der für die Implantation erforderliche Schnitt in die Hornhaut umso schneller heilt, je kürzer er ausgeführt wird - eine minimale Inzision kann sich sogar ventilartig ohne jede Naht spontan verschließen - werden moderne Hinterkammerlinsen, wie beispielsweise in der US 5,716,403 A vorgeschlagen, aus flexiblen Materialien gefertigt. Diese Hinterkammerlinse weist zwei faltbare Haptiken auf, die in einem Anschlussbereich um eine zu der optischen Achse der Hinterkammerlinse senkrechte Faltachse faltbar sind. Die Haptiken dieser Hinterkammerlinse weisen im Wesentlichen starre Stützfüße auf, mittels derer der Linsenkörper in dem Kapselsack abstützbar ist.

Vor Implantation der Hinterkammerlinse aus der US 5,197,981 A wird eine Haptik auf den Linsenkörper gefaltet und in diesen - wenigstens ansatzweise - eingerollt. Wird die Hinterkammerlinse in diesem Zustand gekühlt, so entfaltet sie sich zunächst nicht, sondern kann beispielsweise mit einer Pinzette an der zweiten Haptik gegriffen und durch eine minimale Inzision - entsprechend dem Durchmesser der mit der Haptik aufgerollten Linse - in die Vorderkammer eingeführt werden. Durch die Körperwärme wird die Kühlung der Hinterkammerlinse aufgehoben, der Linsenkörper und die Haptik entfalten sich.

Alternativ ist es auch - beispielsweise aus der EP 1 075 826 A2 - bekannt, eine faltbare Hinterkammerlinse mittels einer dort beschriebenen Vorrichtung zu falten und mittels einer Art Kanüle in den Kapselsack einzubringen, wo sie sich unmittelbar nach Austreten aus dem Öffnungsquerschnitt entfaltet.

Nach dem Einbringen der bekannten gefalteten Hinterkammerlinse in den Kapselsack läuft der Vorgang des Entfaltens im Wesentlichen unkontrolliert ab. Hierbei können in ungünstigen Konstellationen Reizungen der an dem Kapselsack ansetzenden Muskelfasern oder der Iris hervorgerufen werden. Der Operateur korrigiert nach dem Einbringen und Entfalten die Position der Hinterkammerlinse derart, dass die Hinterkammerlinse mittels der Stützfüße in dem Kapselsack abgestützt und ihre sichere Fixierung gewährleistet ist. Eine Hinterkammerlinse gemäß dem Oberbegriff des Anspruchs 1 ist aus der US-A-5 653 754 bekannt.

### Aufgabe

Der Erfindung liegt die Aufgabe zugrunde, eine kapselsackfixierte Hinterkammerlinse vorzuschlagen, bei deren Positionierung im Kapselsack Reizungen des Auges wirksam vermieden werden.

### Lösung

Ausgehend von den bekannten Hinterkammerlinsen wird diese Aufgabe erfindungsgemäß durch die technischen Merkmale des Anspruchs 1 gelöst.

Die Haptik einer erfindungsgemäßen Hinterkammerlinse besteht vorzugsweise aus einem Thermoplast, wie zum Beispiel aus einem Polycarbonat- oder Polyurethan-Harz, aus einem Gemisch dieser beiden oder mit Silikon. Bevorzugt ist das Material der erfindungsgemäßen Hinterkammerlinse hydrophob. Darüber hinaus kann das Material derart modifiziert sein, dass es bestimmte Strahlungsanteile - beispielsweise ultraviolette Strahlung - absorbiert. Auch die Verwendung eingefärbten Materials - beispielsweise zur Farbkorrektur oder um die Position der Linse im Auge leichter kontrollieren zu können - ist möglich.

Durch die Klappbereiche an den Haptiken der erfindungsgemäßen Hinterkammerlinsen treten bei deren Implantation auch punktuell keine hohen Kräfte auf, die zu einer Verletzung des Auges führen könnten. Kommt ein Stützfuß einer erfindungsgemäßen Hinterkammerlinse im Kapselsack mit Körpergewebe in Kontakt, so setzt er dieses nur minimalem Druck aus, da er auf den Linsenkörper zu klappt. Die Federelemente bewirken ein langsames und gleichförmiges Entfalten der Haptiken und deren sichere Anlage in dem Kapselsack ohne zusätzliche Reizung des Auges.

Der Klappbereich der erfindungsgemäßen Hinterkammerlinse ist vorzugsweise ein Filmscharnier. Als Filmscharnier kann insbesondere jeder Bereich eines elastischen Bauteils wirken, der gegenüber benachbarten Bereichen entlang einer Klappachse geschwächt ist.

An einem T-Profil kann beispielsweise durch einen bis zu dem Querbalken reichenden Einschnitt in den Steg ein Filmscharnier ausgebildet sein.

An einer erfindungsgemäßen Hinterkammerlinse kann ein solches Filmscharnier mit parallel zur optischen Achse der Linse verlaufender Klappachse besonders einfach durch einen Einschnitt in einen dem Linsenkörper zugewandten, in der Hauptebene liegenden Steg an einem Stützfuß realisiert werden. Wird dieser Einschnitt lediglich als Schwächung des Stegs ausgeführt, so wirkt die in dem Steg verbleibende Membran zugleich als Federelement, mittels dessen der Stützfuß in der Hauptebene von dem Linsenkörper weg klappbar ist.

Das Federelement einer erfindungsgemäßen Hinterkammerlinse weist bevorzugt eine gegenüber der Dicke eines in der Hauptebene verlaufenden Steges verdünnte Membran auf. Ein derartiges Membranfederelement ist einfach zu fertigen, weil es aus dem Material des Steges bestehen kann. Die Membran kann beispielsweise in der Hauptebene dreieckförmig ausgebildet sein und auf einen linienförmig parallel zu der optischen Achse verlaufenden Klappbereich spitz zulaufen. Alternativ kann die Membran auch in der Hauptebene im Wesentlichen rechteckig ausgebildet sein und entlang eines flächig ausgedehnten Klappbereiches verlaufen.

In einer erfindungsgemäßen Hinterkammerlinse mit dreieckförmig-spitz verlaufender Membran und linienförmiger Klappachse ist der Stützfuß an Bewegungen aus der Hauptebene heraus stärker gehindert, als in einer erfindungsgemäßen Hinterkammerlinse mit rechteckiger, entlang eines flächig ausgedehnten Klappbereiches verlaufender Membran mit vergleichbaren Abmessungen. Durch Variation der Breite des Klappbereiches in der Hauptebene kann die Klappbarkeit des Stützfußes aus der Hauptebene heraus sowie - in Maßen - seine Tordierbarkeit eingestellt werden.

Besonders bevorzugt weist ein Federelement einer erfindungsgemäßen Hinterkammerlinse eine Mehrzahl von Membranen auf, wobei benachbarte Membranen gegen einander in Richtung der optischen Achse versetzt und durch Federstege verbunden sind

Eine erfindungsgemäße Hinterkammerlinse weist bevorzugt mindestens eine faltbare Haptik auf, die zur Implantation der Hinterkammerlinse in ihrem Anschlussbereich um eine in der Hauptebene des Linsenkörpers liegende Faltachse faltbar ist. Die Faltung einer solchen Hinterkammerlinse ist deutlich erleichtert.

Vorzugsweise wird eine erfindungsgemäße Hinterkammerlinse in einem Arbeitsgang gefertigt. Hierdurch wird zusätzlicher Montageaufwand bei der Herstellung der Hinterkammerlinse vermieden und der Prozess der Herstellung beschleunigt. Gegenüber einem umspritzten Linsenkörper oder einer anderweitig zwei- oder mehrteilig montierten Hinterkammerlinse wird das Risiko vermindert, dass Teile der erfindungsgemäßen Hinterkammerlinse unter dem Einfluss des in dem Auge befindlichen Kammerwassers von einander gelöst werden.

Besonders bevorzugt wird die erfindungsgemäße Hinterkammerlinse im Spritzprägeverfahren gefertigt. Alternativ kann auch ein Spritzgussverfahren zur Anwendung kommen. Durch die direkte Formgebung aus der Schmelze können insbesondere Relaxationseffekte vermieden werden, die beispielsweise bei warmverformten, extrudierten Fadenhaptiken typischer Weise zu einem Nachlassen der Fixierungskräfte nach der Implantation führen.

In einer vorteilhaften Ausführung weisen die Stützfüße der erfindungsgemäßen Hinterkammerlinse eine gegenüber ihrer Dicke verbreiterte Stützfläche auf. Die verbreiterte Stützfläche bewirkt eine Verteilung der auf den Kapselsack ausgeübten Kräfte über eine größere Fläche und damit - gegenüber einem Stützfuß mit konstanter Dicke - eine Minderung des ausgeübten Drucks. Beispielsweise können die Stützfüße ein T-förmiges Profil aufweisen. Aber auch die Ausbildung mit einem tropfenförmigen Profil ist denkbar.

### Ausführungsbeispiel

Die Erfindung wird nachfolgend anhand eines Ausführungsbeispiels erläutert. Es zeigen
- Fig. 1a: eine erfindungsgemäße Hinterkammerlinse,
- Fig. 1b: einen ersten Schnitt durch das Federelement dieser Hinterkammerlinse,
- Fig. 1c: einen anderen Schnitt durch dasselbe,
- Fig. 1d: eine perspektivische Detailansicht desselben,
- Fig. 2: geklappte Positionen eines Stützfußes dieser Hinterkammerlinse,
- Fig. 3a: eine zweite erfindungsgemäße Hinterkammerlinse,
- Fig. 3b: eine Detailansicht des Federelements dieser Hinterkammerlinse,
- Fig. 4a: einen ersten Schritt der Faltung dieser Hinterkammerlinse,
- Fig. 4b: einen zweiten Schritt der Faltung derselben,
- Fig. 4c: den Endzustand der Faltung derselben,
- Fig. 5: eine dritte erfindungsgemäße Hinterkammerlinse,
- Fig. 6a: einen Schnitt durch das Federelement einer vierten erfindungsgemäßen Hinterkammerlinse und
- Fig. 6b: eine perspektivische Detailansicht desselben.

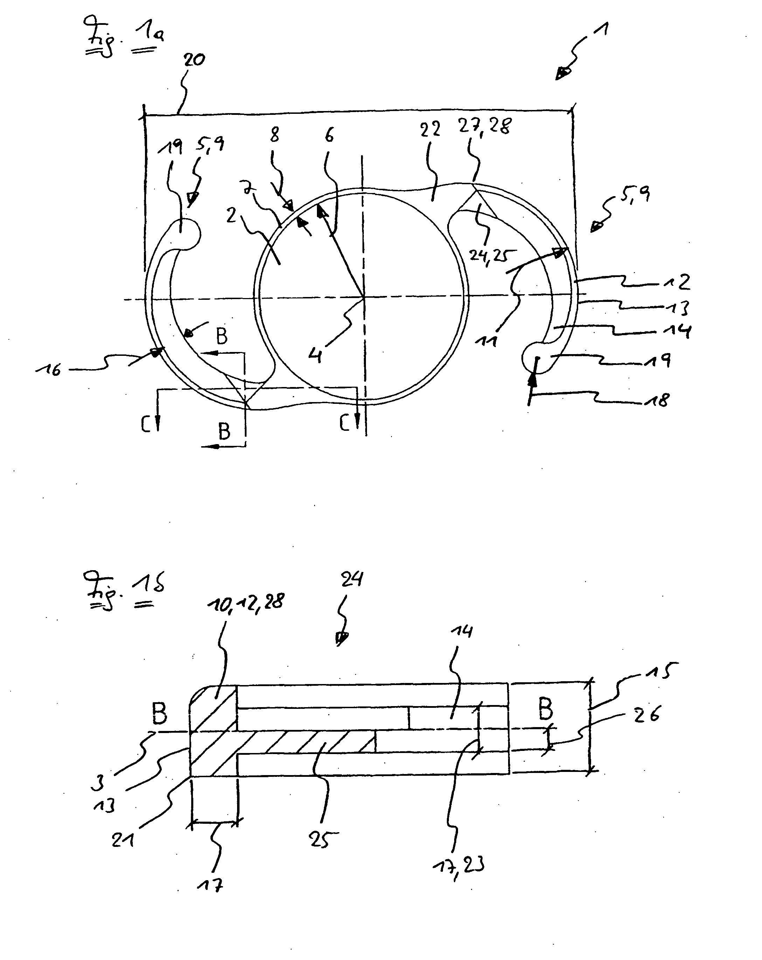

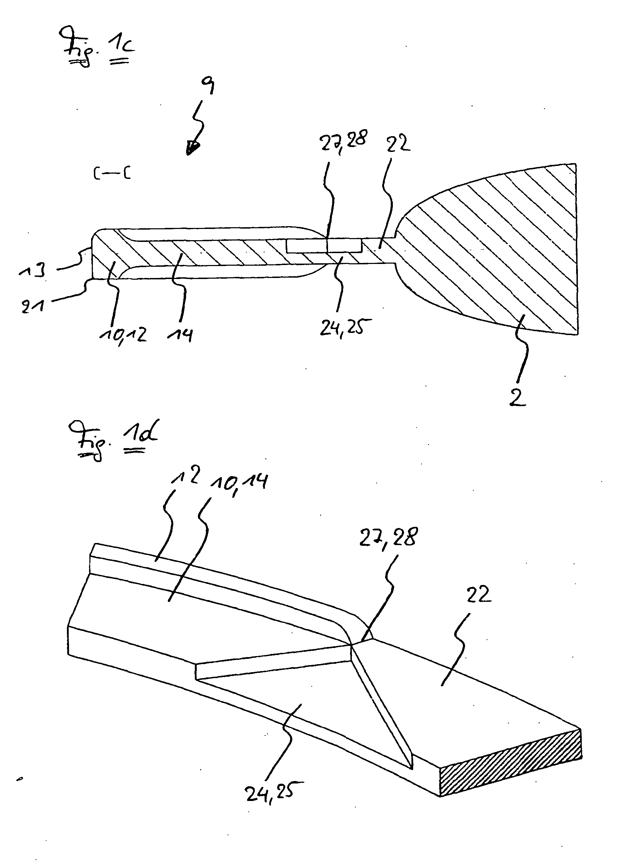

Die in Figur 1a dargestellte **Hinterkammerlinse** 1 weist einen **Linsenkörper** 2 mit (hier in der Bildebene liegender) **Hauptebene** 3 und hierzu senkrechter **optischer Achse** 4 auf. An den Linsenkörper 2 schließen punktsymmetrisch zu seiner optischen Achse 4 zwei in der Hauptebene 3 liegende, C-förmige **Haptiken** 5 an. Die Hinterkammerlinse 1 besteht aus einem Polycarbonat-Polyurethan-Harz, ist im Spritzprägeverfahren in einem Arbeitsgang gefertigt und wird daher als "einstückige" Intraokularlinse bezeichnet. Der nicht dargestellte Prägestempel weist einen Radius von 15 mm auf.

Der Linsenkörper 2 weist einen **Radius** 6 von 3 mm und zusätzlich einen umlaufenden **Rand** 7 mit einer **Breite** 8 von 0,15 mm auf. Die dargestellte Hinterkammerlinse 1 ist damit speziell für die humanmedizinische Anwendung geeignet.

Die Haptiken 5 weisen jeweils einen **Stützfuß** 9 auf, der ein **T-Profil** 10 aufweist. Dies ist insbesondere in den Figuren 1b und 1c verdeutlicht: Figur 1b entspricht einer Schnittansicht gemäß der Schnittlinie B-B, Figur 1c gemäß der Schnittlinie C-C aus Figur 1a. Die Stützfüße 9 sind in der Hauptebene 3 C-förmig mit einem **Radius** 11 von etwa 3 mm gebogen. Auf dem **Querbalken** 12 des T-Profils 10 ist eine radial nach Außen weisende **Stützfläche** 13 ausgebildet, der nach radial nach Innen weisende **Steg** 14 versteift den Stützfuß 9 gegen Biegung in der Hauptebene 3. Der Querbalken 12 weist eine **Höhe** 15 von 0,4 mm, der Steg 14 eine **Höhe** 16 von 0,6 mm auf. Querbalken 12 und Steg 14 weisen eine **Dicke** 17 von 0,2 mm auf. Die Stützfüße 9 enden jeweils in einem auf die Höhe 15 des Querbalkens 12 verdickten und mit einem **Radius** 18 von etwa 0,5 mm verrundeten **Kopf** 19. Die Stützflächen 13 der Stützfüße 9 weisen einen **Abstand** 20 von 12,5 mm auf und stehen im nicht dargestellten implantierten Zustand großflächig mit weitgehend konstanter, niedriger Flächenpressung im Kontakt mit dem Kapselsack. So wird einerseits das Risiko der Nekrotisierung des anliegenden Gewebes vermieden, andererseits eine gute Fixierung der Hinterkammerlinse 1 gewährleistet.

Der Querbalken 12 der Stützfüße 9 weist proximal scharfe **Kanten** 21 auf, die im nicht dargestellten implantierten Zustand der Hinterkammerlinse 1 durch die natürliche Bewegung ein Abschaben von zellulären Belägen auf dem anliegenden Kapselsack bewirken.

Die Stützfüße 9 sind jeweils in einem dreieckförmigen **Anschlussbereich** 22 der Haptik 5 mit dem Linsenkörper 2 verbunden. Die Anschlussbereiche 22 weisen eine - wiederum in den Figuren 1b und 1c dargestellte - **Dicke** 23 von 0,2 mm auf. Durch die gegenüber dem Querbalken 12 des Stützfußes 9 geschwächte Gestaltung ist die dargestellte Hinterkammerlinse 1 in den Anschlussbereichen 22, nicht aber in den Stützfüßen 9 um eine nicht dargestellte Achse in der Hauptebene 3 faltbar. Den Übergang zwischen Anschlussbereich 22 und Stützfuß 9 bildet ein - perspektivisch in Figur 1d dargestelltes - **Federelement** 24, das durch eine dreieckförmige **Membran** 25 gegenüber der Dicke 17 des Steges 14 auf eine **Dicke** 26 von 0,1 mm verdünnt ist. Die Membran 25 läuft auf einen linienförmigen **Klappbereich** 27 spitz zu. Der Querbalken 12 des Stützfußes 9 bildet in dem Klappbereich 27 ein **Filmscharnier** 28.

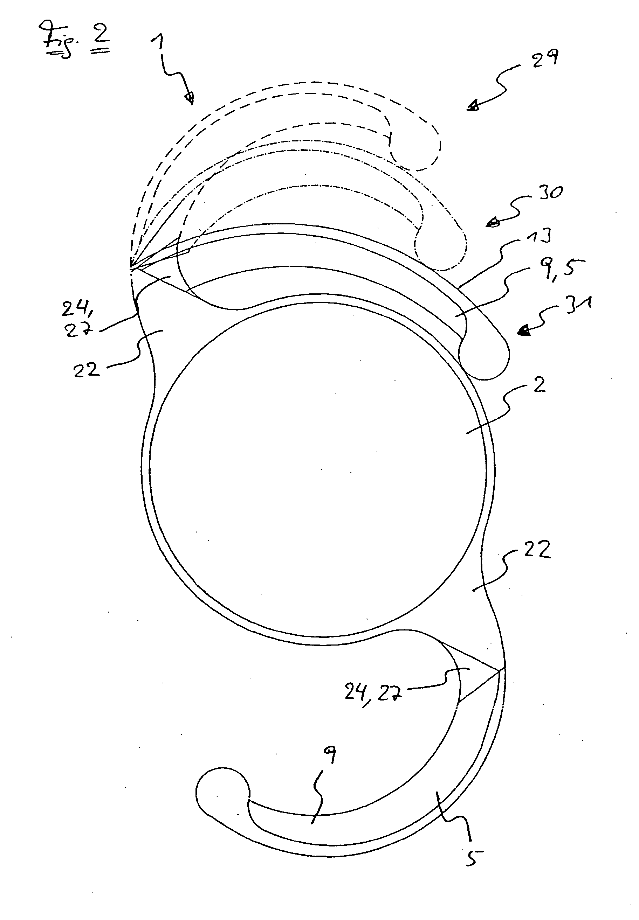

Figur 2 zeigt einen Stützfuß 9 der Hinterkammerlinse 1 in der **Grundposition** 29 (gestrichelt), in einer leicht angeklappten **Zwischenposition** 30 (strichpunktiert) und in einer **Endstellung** 31 (durchgezogen). Ist der Stützfuß 9 in dem Klappbereich 27 aus der Grundposition 29 in Richtung auf die Endstellung 31 auf den Linsenkörper 2 zu geklappt, so wird die Membran 25 in nicht dargestellter Weise elastisch verformt und aufgeworfen und bewirkt eine in der Hauptebene 3 von dem Linsenkörper 2 weg in Richtung der Grundposition 29 weisende rückstellende Kraft auf den Stützfuß 9. In der dargestellten Endstellung 31 wird der Stützfuß 9 durch eine radial auf die Stützfläche 13 wirkende Kraft an den Linsenkörper 2 gedrückt und gegenüber der Zwischenposition 30 zusätzlich elastisch verformt.

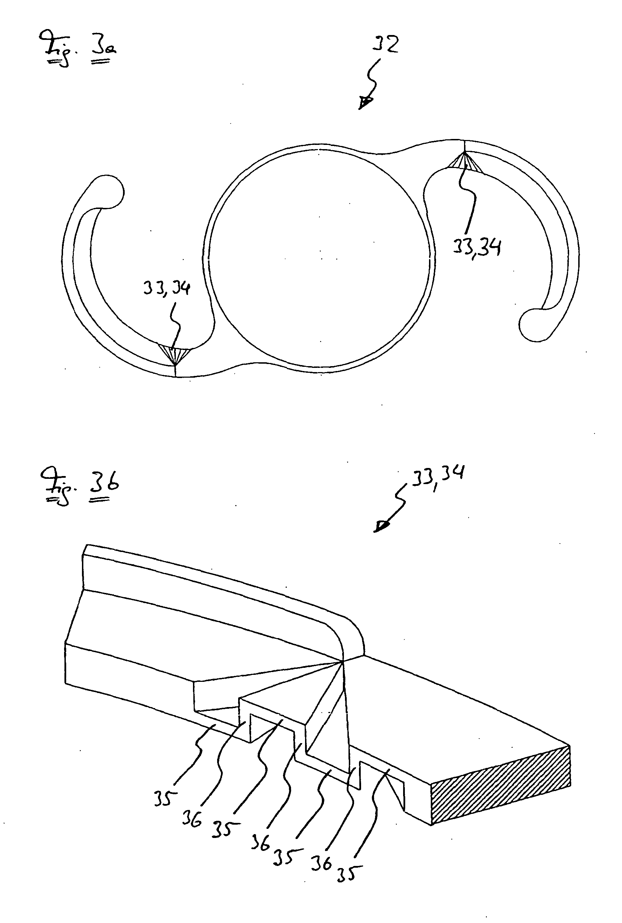

Figur 3a zeigt eine zweite erfindungsgemäße **Hinterkammerlinse** 32, die sich von der ersten, in den vorhergegangenen Figuren dargestellten Hinterkammerlinse 1 lediglich durch ein anderes **Federelement** 33 unterscheidet, das im Detail in Figur 3b dargestellt ist. Dieses Federelement 33 ist gleichfalls von einem linienförmigen **Klappbereich** 34 ausgehend dreieckig, weist aber vier wechselweise in Richtung der optischen Achse 3 gegen einander versetzte **Membranen** 35 auf, die sternförmig geradlinig von dem Klappbereich 34 ausgehen und die jeweils durch einen **Federsteg** 36 verbunden sind. Durch die "Zickzackform" ist die nicht dargestellte Faltung dieses Federelements 33 beim Anklappen des Stützfußes 9 einerseits definiert und andererseits gegenüber dem Federelement 24 der zuvor dargestellten Hinterkammerlinse 1 vergleichmäßigt, weil spontane Änderungen der Faltung bei Änderungen der Belastung vermieden werden.

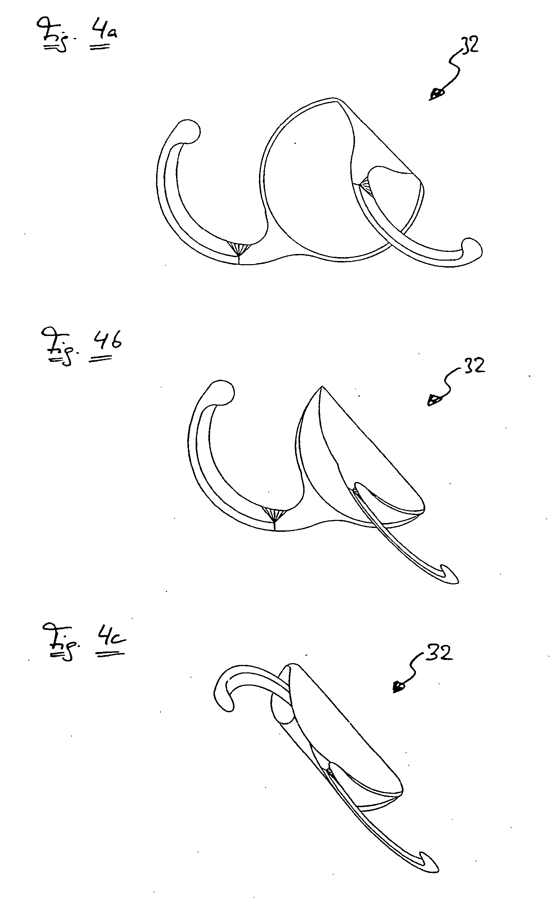

Die Figuren 4a bis 4c zeigen am Beispiel der Hinterkammerlinse 32 gemäß den Figuren 3a und 3b drei Schritte der Faltung dieser Hinterkammerlinse 32 bis hin zum Endzustand, in dem die Hinterkammerlinse 32 für die Implantation bereit ist.

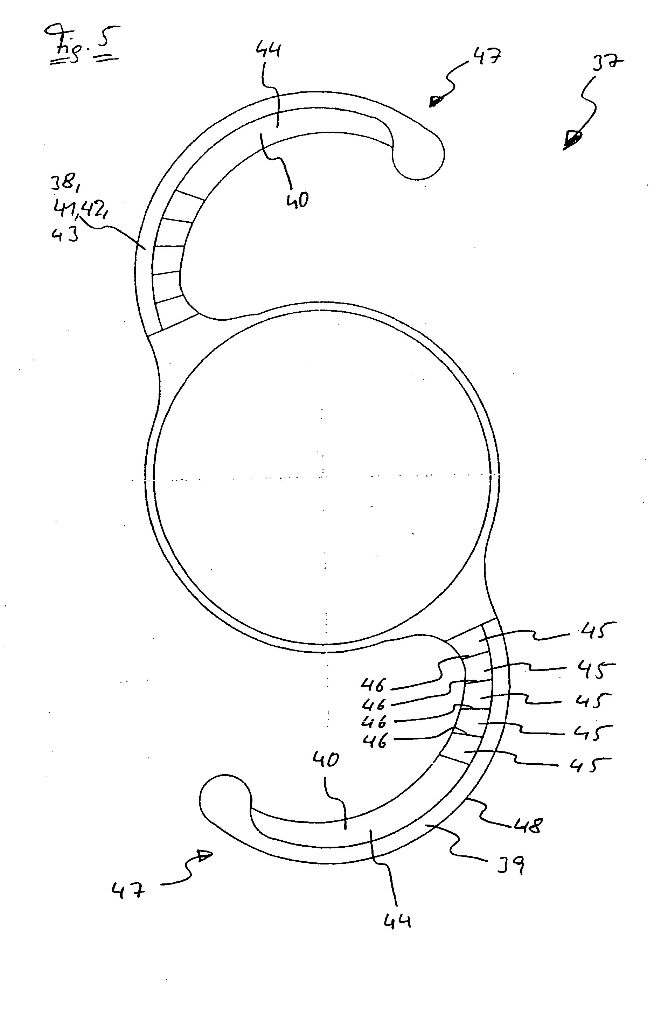

Figur 5 zeigt eine dritte erfindungsgemäße Hinterkammerlinse 37, die sich wiederum von den zuvor dargestellten Hinterkammerlinsen 1, 32 durch ein alternativ gestaltetes **Federelement** 38 unterscheidet. Auch hier bildet der **Querbalken** 39 der Haptik 5 hinter einer Schwächung des **Steges** 40 ein **Filmscharnier** 41. Dieses Filmscharnier 41 erstreckt sich jedoch in einem **Bogenabschnitt** 42 des Steges von etwa 2,5 mm über einen flächig ausgedehnten **Klappbereich** 43, in dem der **Stützfuß** 44 dieser Hinterkammerlinse 37 klappbar ist.

Das Federelement 38 weist fünf im Wesentlichen rechteckige, wiederum in Richtung der optischen Achse 4 gegen einander versetzte **Membranen** 45 auf, die gleichfalls jeweils durch einen **Federsteg** 46 verbunden sind.

Gegenüber den zuvor dargestellten Hinterkammerlinsen 1, 32 sind die **Haptiken** 45 der Hinterkammerlinse 37 gemäß Figur 5 in dem Klappbereich 43 zusätzlich - in Maßen - tordierbar. Außerdem ist die Spannung in der **Stützfläche** 48 im nicht dargestellten geklappten Zustand deutlich reduziert.

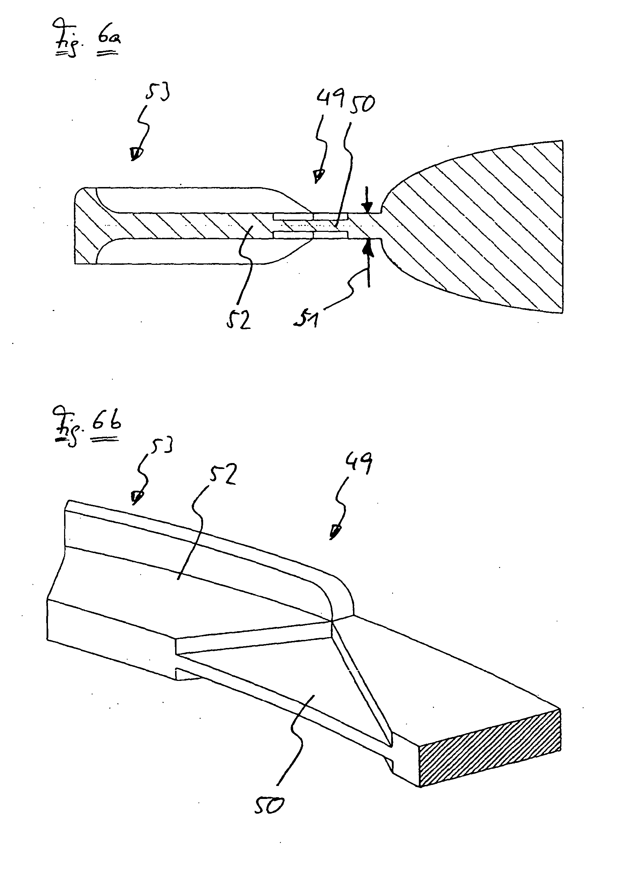

Die Figuren 6a und 6b zeigen zwei Ansichten einer nicht weiter dargestellten vierten erfindungsgemäßen Hinterkammerlinse entsprechend den Ansichten in den Figuren 1c und 1d. Diese vierte Hinterkammerlinse weist wiederum eine alternativ gestaltetes **Federelement** 49 auf. Dieses weist wie das Federelement 24 der Hinterkammerlinse 1 eine dreieckförmige **Membran** 50 auf, die gegenüber der **Dicke** 51 des **Steges** 52 auf die Hälfte verdünnt ist. Im Gegensatz zum Federelement 24 der Hinterkammerlinse 1 ist der Steg 52 an der hier dargestellten Hinterkammerlinse jedoch proximal und distal gleichmäßig geschwächt. Hierdurch wird ein gleichmäßigeres Klappen des **Stützfußes** 53 in der hier nicht dargestellten Hauptebene erreicht.

In den Figuren sind
- 1: Hinterkammerlinse
- 2: Linsenkörper
- 3: Hauptebene
- 4: optische Achse
- 5: Haptik
- 6: Radius
- 7: Rand
- 8: Breite
- 9: Stützfuß
- 10: T-Profil
- 11: Radius
- 12: Querbalken
- 13: Stützfläche
- 14: Steg
- 15: Höhe
- 16: Höhe
- 17: Dicke
- 18: Radius
- 19: Kopf
- 20: Abstand
- 21: Kante
- 22: Anschlussbereich
- 23: Dicke
- 24: Federelement
- 25: Membran
- 26: Dicke
- 27: Klappbereich
- 28: Filmscharnier
- 29: Grundposition
- 30: Zwischenposition
- 31: Endstellung
- 32: Hinterkammerlinse
- 33: Federelement
- 34: Klappbereich
- 35: Membran
- 36: Federsteg
- 37: Hinterkammerlinse
- 38: Federelement
- 39: Querbalken
- 40: Steg
- 41: Filmscharnier
- 42: Bogenabschnitt
- 43: Klappbereich
- 44: Stützfuß
- 45: Membran
- 46: Federsteg
- 47: Haptik
- 48: Stützfläche
- 49: Federelement
- 50: Membran
- 51: Dicke
- 52: Steg
- 53: Stützfuß

## Patentansprüche

1. Hinterkammerlinse (1, 32, 37), die anstelle einer natürlichen Linse eines Auges in einen Kapselsack der Linse derart implantierbar ist, dass eine optische Achse (4) der Hinterkammerlinse (1, 32, 37) eine Netzhaut des Auges trifft, wobei die Hinterkammerlinse (1, 32, 37) einen Linsenkörper (2) mit einer zu der optischen Achse (4) senkrechten Hauptebene (3) und mehrere Haptiken (5, 45) aufweist, die jeweils in einem Anschlussbereich (22) an den Linsenkörper (2) anschließen und die mittels im Wesentlichen starrer, in der Hauptebene (3) von dem Linsenkörper (2) abstehender Stützfüße (9, 44) dessen Abstützung in dem Kapselsack gewährleisten, wobei jeweils zwischen Anschlussbereich (22) und Stützfuß (9, 44, 52) der Haptiken (5, 45) ein Klappbereich (27, 34, 43) angeordnet ist, in dem der Stützfuß (9, 44, 52) in der Hauptebene (3) klappbar ist, ***gekennzeichnet durch*** ein Federelement (24, 33, 38, 49), mittels dessen der auf den Linsenkörper (2) zu geklappte Stützfuß (9, 44, 52) in der Hauptebene (3) von dem Linsenkörper (2) weg rückstellbar ist.

2. Hinterkammerlinse (1, 32, 37) nach dem vorgenannten Anspruch, ***dadurch gekennzeichnet, dass*** der Klappbereich (27, 34, 43) ein Filmscharnier (28, 41) ist.

3. Hinterkammerlinse (1, 32, 37) nach einem der vorgenannten Ansprüche, ***dadurch gekennzeichnet, dass*** das Federelement (24, 33, 38, 49) eine gegenüber der Dicke (17, 51) eines in der Hauptebene (3) verlaufenden Steges (14, 40, 52) verdünnte Membran (25, 35, 45, 50) aufweist.

4. Hinterkammerlinse (1, 32) nach dem vorgenannten Anspruch, ***dadurch gekennzeichnet, dass*** die Membran (25, 35, 50) in der Hauptebene (3) dreieckförmig ausgebildet ist und auf einen linienförmig parallel zu der optischen Achse (4) verlaufenden Klappbereich (27, 34) spitz zuläuft.

5. Hinterkammerlinse (37) nach Anspruch 3, ***dadurch gekennzeichnet, dass*** die Membran (45) in der Hauptebene (3) im Wesentlichen rechteckig ausgebildet ist und entlang eines flächig ausgedehnten Klappbereiches (43) verläuft.

6. Hinterkammerlinse (32, 37) nach einem der Ansprüche 4 und 5, ***dadurch gekennzeichnet, dass*** das Federelement (33, 38) eine Mehrzahl von Membranen (35, 45) aufweist, wobei benachbarte Membranen (35, 45) gegen einander in Richtung der optischen Achse (3) versetzt und durch Federstege (36, 46) verbunden sind.

7. Hinterkammerlinse (1, 32, 37) nach einem der vorgenannten Ansprüche, ***dadurch gekennzeichnet, dass*** die Hinterkammerlinse (1, 32, 37) mindestens eine faltbare Haptik (5, 45) aufweist, die zur Implantation der Hinterkammerlinse (1, 32, 37) in ihrem Anschlussbereich (22) um eine in der Hauptebene (3) des Linsenkörpers (2) liegende Faltachse faltbar ist.

8. Hinterkammerlinse (1, 32, 37) nach einem der vorgenannten Ansprüche, ***dadurch gekennzeichnet, dass*** die Hinterkammerlinse (1, 32, 37) in einem Arbeitsgang gefertigt ist.

9. Hinterkammerlinse (1, 32, 37) nach dem vorgenannten Anspruch, ***dadurch gekennzeichnet, dass*** die Hinterkammerlinse (1, 32, 37) im Spritzprägeverfahren gefertigt ist.

10. Hinterkammerlinse (1, 32, 37) nach einem der vorgenannten Ansprüche, ***dadurch gekennzeichnet, dass*** die Stützfüße (9, 44) eine gegenüber ihrer Dicke verbreiterte Stützfläche (13, 48) aufweisen.

11. Hinterkammerlinse (1, 32, 37) nach dem vorgenannten Anspruch, ***dadurch gekennzeichnet, dass*** die Stützfüße (9, 44) ein T-Profil (10) aufweisen.

## Claims

1. A posterior chamber lens (1, 32, 37) which can be implanted in a capsule bag of the lens instead of a natural lens of an eye in such a way that an optical axis (4) of the posterior chamber lens (1, 32, 37) meets a retina of the eye, with the posterior chamber lens (1, 32, 37) comprising a body (2) of the lens with a principal plane (3) perpendicular to the optical axis (4) and several haptics (5, 45) which are each adjacent to the body (2) of the lens in a connecting region (22) and which ensure its support in the capsule bag by means of substantially rigid supporting feet (9, 44) protruding in the principal plane (3) from the body (2) of the lens, and a folding region (27, 34, 43) each which is arranged between the connecting region (22) and the supporting foot (9, 44, 52) of the haptics (5, 45) and in which the supporting foot (9, 44, 52) can be folded in the principal plane (3), **characterized by** a spring element (24, 33, 38, 49) by means of which the supporting foot (9, 44, 52) folded down onto the body (2) of the lens can be returned in the principal plane (3) away from the body (2) of the lens.

2. A posterior chamber lens (1, 32, 37) according to the preceding claim, **characterized in that** the folding region (27, 34, 43) is a film hinge (28, 41).

3. A posterior chamber lens (1, 32, 37) according to one of the preceding claims, **characterized in that** the spring element (24, 33, 38, 49) comprises a membrane (25, 35, 45, 50) which is thinned relative to the thickness (17, 51) of a web (14, 40, 52) extending in the principal plane (3).

4. A posterior chamber lens (1, 32) according to the preceding claim, **characterized in that** the membrane (25, 35, 50) is provided with a triangular configuration in the principal plane (3) and tapers towards a folding region (27, 34) extending in the form of a line parallel to the optical axis (4).

5. A posterior chamber lens (37) according to claim 3, **characterized in that** the membrane (45) in the principal plane (3) is provided with a substantially rectangular configuration and extends along a planar extended folding region (43).

6. A posterior chamber lens (32, 37) according to one of the claims 4 and 5, **characterized in that** the spring element (33, 38) comprises a plurality of membranes (35, 45), with adjacent membranes (35, 45) are mutually offset in the direction of the optical axis (4) and are joined by spring webs (36, 46).

7. A posterior chamber lens (1, 32, 37) according to one of the preceding claims, **characterized in that** the posterior chamber lens (1, 32, 37) comprises at least one foldable haptic (5, 45) which can be folded for the implantation of the posterior chamber lens (1, 32, 37) in its connecting region (22) about a folding axis situated in the principal plane (3) of the body (2) of the lens.

8. A posterior chamber lens (1, 32, 37) according to one of the preceding claims, **characterized in that** the posterior chamber lens (1, 32, 37) is made in one working cycle.

9. A posterior chamber lens (1, 32, 37) according to the preceding claim, **characterized in that** the posterior chamber lens (1, 32, 37) is made with the injection embossing method.

10. A posterior chamber lens (1, 32, 3 7) according to one of the preceding claims, **characterized in that** the supporting feet (9, 44) have a supporting surface (13, 48) which is widened with respect to its thickness.

11. A posterior chamber lens (1, 32, 37) according to the preceding claim, **characterized in that** the supporting feet (9, 44) have a T-profile (10).

## Revendications

1. Lentille de chambre postérieure (1, 32, 37) pouvant être implantée à la place d'un cristallin naturel d'un oeil dans un sac capsulaire du cristallin de telle sorte qu'un axe optique (4) de la lentille de chambre postérieure (1, 32, 37) atteigne une rétine de l'oeil, la lentille de chambre postérieure (1, 32, 37) possédant un corps de lentille (2) avec un plan principal (3) perpendiculaire à l'axe optique (4) et plusieurs haptiques (5, 45) qui se raccordent chacun dans une zone de raccordement (22) au corps de lentille (2) et qui garantissent, au moyen de pieds d'appui (9, 44) rigides dépassant du corps de lentille (2) dans le plan principal (3), l'appui de celui-ci dans le sac capsulaire, une zone de repliement (27, 34, 43), dans laquelle le pied d'appui (9, 44, 52) peut être rabattu dans le plan principal (3), étant disposée entre la zone de raccordement (22) et le pied d'appui (9, 44, 52) des haptiques (5, 54), **caractérisée en ce qu'**elle comprend un élément élastique (24, 33, 38, 49) au moyen duquel le pied d'appui (9, 44, 52) rabattu vers le corps de lentille (2) peut être écarté du corps de lentille (2) et ramené dans le plan principal (3).

2. Lentille de chambre postérieure (1, 32, 37) selon la revendication précédente, **caractérisée en ce que** la zone de repliement (27, 34, 43) est une charnière à film (28, 41).

3. Lentille de chambre postérieure (1, 32, 37) selon l'une quelconque des revendications précédentes, **caractérisée en ce que** l'élément élastique (24, 33, 38, 49) présente une membrane (25, 35, 45, 50) amincie par rapport à l'épaisseur (17, 51) d'une barrette (14, 40, 52) passant dans le plan principal (3).

4. Lentille de chambre postérieure (1, 32) selon la revendication précédente, **caractérisée en ce que** la membrane (25, 35, 50) est de forme triangulaire dans le plan principal (3) et s'amincit en pointe vers une zone de repliement (27, 34) en forme de ligne parallèle à l'axe optique (4).

5. Lentille de chambre postérieure (37) selon la revendication 3, **caractérisée en ce que** la membrane (45) est pour l'essentiel rectangulaire dans le plan principal (3) et s'étend le long d'une zone de repliement (43) étendue en surface.

6. Lentille de chambre postérieure (32, 37) selon l'une quelconque des revendications 4 et 5, **caractérisée en ce que** l'élément élastique (33, 38) possède une pluralité de membranes (35, 45), les membranes voisines (35, 45) étant décalées l'une par rapport à l'autre en direction de l'axe optique (4) et reliées par des barrettes élastiques (36, 46).

7. Lentille de chambre postérieure (1, 32, 37) selon l'une quelconque des revendications précédentes, **caractérisée en ce que** la lentille de chambre postérieure (1, 32, 37) possède au moins un haptique (5, 45) repliable, qui peut être replié dans sa zone de raccordement (22) autour d'un axe de pliage situé dans le plan principal (3) du corps de lentille (2) en vue de pour l'implantation de la lentille de chambre postérieure (1, 32, 37).

8. Lentille de chambre postérieure (1, 32, 37) selon l'une quelconque des revendications précédentes, **caractérisée en ce que** la lentille de chambre postérieure (1, 32, 37) est fabriquée en une seule étape de travail.

9. Lentille de chambre postérieure (1, 32, 37) selon l'une quelconque des revendications précédentes, **caractérisée en ce que** la lentille de chambre postérieure (1, 32, 37) est fabriquée par injection-compression.

10. Lentille de chambre postérieure (1, 32, 37) selon l'une quelconque des revendications précédentes, **caractérisée en ce que** les pieds d'appui (9, 44) ont une surface d'appui (13, 48) élargie par rapport à leur épaisseur.

11. Lentille de chambre postérieure (1, 32, 37) selon la revendication précédente, **caractérisée en ce que** les pieds d'appui (9,44) ont un profit en forme de T (10).
